# EUROPÄISCHE PATENTSCHRIFT

(11) **EP 2 072 085 B1**
(45) Veröffentlichungstag und Bekanntmachung des Hinweises auf die Patenterteilung: **16.10.2013**
(21) Anmeldenummer: 08162722.6
(22) Anmeldetag: 21.08.2008
(51) Int. Cl.: A61Q 19/10, A61Q 9/02, A61K 8/44, A61K 8/46, A61K 8/81, A61K 8/86, A61K 8/39

(54) **Rasierduschzubereitung**
Shaving shower preparation
Préparation de douche de rasage

(30) Priorität: 20.12.2007 DE 102007062427
(43) Veröffentlichungstag der Anmeldung: 24.06.2009
(73) Patentinhaber: Beiersdorf AG, 20245 Hamburg (DE)
(72) Erfinder: Argembeaux, Horst, 21465 Wentorf (DE); Belser, Miriam, 22529 Hamburg (DE); Counradi, Katrin, 22143 Hamburg (DE)

(56) Entgegenhaltungen:
- WO-A-97/25975
- JP-A- 2006 096 754
- US-A- 5 976 520
- US-A- 6 107 352
- US-A1- 2005 158 269
- US-A1- 2005 189 377

## Beschreibung

Zu den täglichen hygienischen Routinen gehört für viele Verbraucherinnen und Verbraucher das Duschen und für die männlichen Verbraucher auch die Barthaarrasur. Das Rasieren bestimmter Körperpartien wie z. B. Beine und Achsel ist aber auch bei Verbraucherinnen und zunehmend auch bei Männern weit verbreitet.

Üblicherweise werden zum Duschen handelsübliche Duschpräparate wie Duschgel, Duschemulsionen oder Duschöle verwendet. Für die Rasur, sofern es sich dabei um eine Nassrasur handelt verwendet man in der Regel aufschäumbare oder selbstschäumende Seifen, Gele oder Schäume zur Vorbereitung des Rasurprozesses mit der Klinge.

Da viele Verbraucher die Rasur in Kombination vor oder nach dem Duschprozess vollziehen, wäre es für diese ein großer Vorteil ein Produkt zur Verfügung zu haben, dass beide Prozesse gleichermaßen unterstützt. Benutzt man jedoch ein Duschbad des Standes der Technik zur Rasur, stellt man fest, dass dieser Prozess sehr unkomfortabel ist. So reicht die Menge Schaum und insbesondere die Stabilität des Schaums nicht aus um diesen auf der Rasurzone zu verteilen und dort bis zum Ende der Rasur zu halten. Die Konsistenz des Schaum ist auch nicht kompakt genug um eine dämpfende Wirkung auf den Rasierer auszuüben, außerdem neigt der Schaum auch dazu schnell abzufließen. Zieht man den Rasierer über die Rasurzone, stellt man ein mangelhaftes Gleiten fest, das zu unangenehmen ziepen führt und im Extremfall zu Schnittverletzungen führen kann.

Benutzt man ein Rasierprodukt zum Duschen, stellt man fest, dass dieses sich wegen des kompakten Schaumes nur schlecht über den ganzen Körper verteilen lässt. Da solche Produkte meist auf Seifenbasis aufgebaut sind, ist deren Verträglichkeit deutlich schlechter als die normaler Duschgels, die Säure-Base-Balance der Haut wird gestört. Dieses kann zu unangenehmen brennen und jucken insbesondere im Bereich der Schleimhäute kommen oder zu Rötungen bis hin zur Bildung von Erythemen. Ein weiterer Nachteil ist deren schlechte Abspülbarkeit von der Haut.

Duschgele mit guten Schaumeigenschaften sind bekannt. So werden z. B. in der DE 10 2005 033 663 Kosmetische Reinigungszubereitungen enthaltend anionische Schwefelsäureester, amphotheres Tensid, ethoxylierte Glycerin-Fettsäureester mit 3 bis 12 EO-Einheiten, wobei der Gehalt an ethoxylierten Glycerin-Fettsäureester mehr als 1,2 % beträgt und der Gehalt an Tensiden insgesamt weniger als 16 Gew.-% beträgt wobei als Tenside auch ethoxylierte Glycerin-Fettsäureester mit 3 bis 12 EO-Einheiten zu verstehen sind, offenbart. Dies sind Duschgele, die bei geringer Gesamttensidkonzentration ein höheren Anteil amphoterer Tenside als anionischer Tenside enthalten und die somit besonders mild sind. Für einen guten Rasurprozess fehlt solchen Schäumen jedoch ein gewisses Haftungsvermögen, darüber hinaus zeigen derartige Produkte keine rasurgeeigneten Gleiteigenschaften.

Dem Stand der Technik mangelt es also an Zubereitungen, die Vorteile eines Duschegels mit denen eines Rasierproduktes vereinen ohne deren Nachteile aufzuweisen.

Es wurde überraschend festgestellt, dass eine kosmetische Reinigungs- und Rasurzubereitung mit einem Tensidsystem aus amphoteren Tensid mit anionischen Tensid und einem Verhältnis von amphoterem zu anionischem Tensid größer 1 den Nachteilen des Stand der Technik abhilft und sich ausgezeichnet als Basis für ein Shower und Shave Produkt eignet.

Es wird ein Tensidsystem aus Cocamidopropylbetain und Laurylethersulfaten verwendet Weiter verbessert wird dieses Tensidsystem durch zusätzliche Anteile bis max. 3 % von ethoxilierten Fettsäureestern, insbesonders Fettsäure-Glycerinestern mit einer Kettenlänge von 10 - 22. Als besonders geeigneter Ester zeigt sich das PEG-7 Glyceryl Cocoate. Derartige Tensidsysteme zeigen ausgezeichnete Schaumeigenschaften. Es ist eine Kombination kationischer Polymere mit nichtionischen Polymeren enthalten. Die Kombination ist Polyquaternium-7 und PEG-90 M. Dadurch lässt sich ein verbessertes Gleitvermögen mit einem kompakten Schaum mit gutem Haftvermögen jedoch ohne klebriger Sensorik erzielen, ohne das fadenziehende Produkte oder klebrige Schäume erhalten werden.

Überraschenderweise wurde auch gefunden, dass derartige Systeme die Schneidkraft von Haaren reduzieren. Im Vergleich zu nicht erfindungsgemäßen Duschgelformulierungen zeigte sich, dass das erfindungsgemäße Produkt die Kraft zur Trennung eines einzelnen Haares mit Hilfe einer Rasierklinge signifikant reduziert.

Die Erfindung umfasst auch die Verwendung eines Tensidsystems wie oben beschrieben mit einer Kombination kationischer Polymere mit nichtionischen Polymeren wie oben beschrieben zur Verbesserung des Rasierer-Gleitvermögens und Schaumhaftvermögens einer kosmetischen Reinigungszubereitung oder auch zur Verbesserung der Reinigungsleistung, Milde, Verteilbarkeit und Abspülbarkeit einer Rasurzubereitung.

Produkte mit den beschriebenen Tensidsystemen und Polymersysteme zeigen eine gute Reinigungsleistung, sind mild, lassen sich gut verteilen und abspülen. Sie haben einen kompakten, gut haftenden Schaum und zeigen ein gutes Gleitvermögen. Mit solchen Produkten behandelte Haut lässt sich ausgesprochen gut rasieren und fühlt sich gepflegt an.

### Beispiele

| | **1** | **2** | **3** | **5** | **6** | **7** |
|---|---|---|---|---|---|---|
| Natrium Laurylethersulfat | 5,5 | 4,5 | 4,0 | 4,5 | 4,0 | 4,5 |
| Cocoamidopropyl Betain | 9,0 | 9,0 | 8.0 | 7,0 | 7,0 | 9,0 |
| PEG-7 Glyceryl Cocoate | 1,0 | 1,5 | 2,0 | 1,5 | 2,5 | 2,0 |
| PEG-90M | 0,05 | 0,1 | 0,05 | 0,075 | 0,03 | 0,1 |
| Polyquaternium-7 | 0,3 | 0,4 | 0,4 | 0,40 | 0,5 | 0,35 |
| PEG-3 Distearate | 1,2 | 1,5 | - | 1,5 | 1,2 | 1,2 |
| PEG-200 hydriertes Glycerylpalmitat | 1,0 | 0,4 | 0,8 | 0,8 | 0,2 | 0,5 |
| PEG-40 hydriertes Rizinusöl | 0,7 | 0,6 | 0,4 | 0,6 | 0,4 | 0,5 |
| Panthenol | - | - | - | - | - | 0,1 |
| Sojaöl | - | 0,2 | - | - | - | - |
| Carica Papaya | - | - | - | - | - | - |
| Natriumchlorid | 0,3 | - | 0,3 | 0,1 | - | - |
| Natriumsalicylat | 0,4 | 0,4 | 0,3 | 0,40 | 0,4 | 0,45 |
| Natriumbenzoat | 0,4 | 0,5 | 0,5 | 0,45 | 0,4 | 0,4 |
| Zitronensäure | q.s. | q.s. | q.s. | q.s. | q.s. | q.s. |
| Parfum | q.s. | q.s. | q.s. | q.s. | q.s. | q.s. |
| Wasser | ad 100 | ad 100 | ad 100 | ad 100 | ad 100 | ad 100 |

## Patentansprüche

1. Kosmetische Reinigungs- und Rasurzubereitung mit einem Tensidsystem aus amphoteren Tensid mit anionischen Tensid und einem Verhältnis von amphoterem zu anionischem Tensid größer 1, **dadurch gekennzeichnet, dass** zusätzlich eine Kombination kationischer Polymere mit nichtionischen Polymeren enthalten ist, wobei als Kombination kationischer Polymere mit nichtionischen Polymeren Polyquaternium-7 und PEG-90 M enthalten sind, dass zusätzlich höchstens 3% von ethoxilierten Fettsäureestern, enthalten sind, wobei als ethoxilierter Fettsäureester PEG-7 Glyceryl Cocoate enthalten ist und dass das Tensidsystem aus Cocamidopropylbetain und Laurylethersulfaten verwendet wird.

2. Verwendung einer Reinigungs- und Rasurzubereitung gemäß Anspruch 1 zur Reduzierung der Schneidkraft von Haaren.

## Claims

1. Cosmetic cleaning and shaving preparation with a surfactant system of amphoteric surfactant with anionic surfactant and a ratio of amphoteric to anionic surfactant greater than 1, **characterized in that** additionally a combination of cationic polymers with nonionic polymers is present, where polyquaternium-7 and PEG-90 M are present as a combination of cationic polymers with nonionic polymers, that additionally at most 3% of ethoxylated fatty acid esters are present, where PEG-7 glycerol cocoate is present as ethoxylated fatty acid ester, and that the surfactant system of cocamidopropylbetaine and lauryl ether sulphates is used.

2. Use of a cleaning and shaving preparation according to Claim 1 for reducing the cutting force of hair.

## Revendications

1. Préparation cosmétique de nettoyage et de rasage contenant un système tensioactif constitué par un agent tensioactif amphotère et un agent tensioactif anionique et un rapport d'agent tensioactif amphotère à agent tensioactif anionique supérieur à 1, **caractérisée en ce qu'**elle contient en outre une combinaison de polymères cationiques et de polymères non ioniques, du Polyquaternium-7 et du PEG-90 M étant contenus comme combinaison de polymères cationiques et de polymères non ioniques, **en ce qu'**elle contient en outre au plus 3% d'esters éthoxylés d'acides gras, du cocoate de PEG-7-glycéryle étant contenu comme ester d'acide gras éthoxylé et **en ce que** le système tensioactif de cocoamidopropylbétaïne et de lauryléthersulfates est utilisé.

2. Utilisation d'une préparation de nettoyage et de rasage selon la revendication 1 pour la réduction de la force de coupe des poils.
